# EUROPEAN PATENT SPECIFICATION

(11) **EP 2 711 129 B1**
(45) Date of publication and mention of the grant of the patent: **06.01.2016**
(21) Application number: 13197889.2
(22) Date of filing: 17.09.2010
(51) Int. Cl.: B24B 19/02, B24B 19/16, B24B 41/06, B21G 1/12, A61M 5/32, A61M 25/06

(54) **Method and apparatus for making a hole, slot and/or depression in a needle proximal to its tip**
Verfahren und Vorrichtung zur Herstellung eines Lochs, eines Schlitzes und/oder einer Vertiefung in der Nähe der Spitze einer Nadel
Procédé et appareil de fabrication d'un trou, fente et/ou dépression dans une aiguille à proximité de sa pointe

(30) Priority: 18.09.2009 IN DE19492009
(43) Date of publication of application: 26.03.2014
(62) Divisional of application: 10770623.6
(73) Proprietor: Poly Medicure Limited, Faridabad, Haryana 121004 (IN)
(72) Inventor: Baid, Rishi, 110048 New Delhi (IN)
(74) Representative: Korenberg, Alexander Tal

(56) References cited:
- EP-A1- 0 591 992
- WO-A1-2004/065036
- WO-A1-2005/087296
- US-A- 4 216 628
- US-A- 5 709 668
- US-A- 5 858 002

## Description

### FIELD OF THE INVENTION

The invention relates to a method and apparatus for making a needle with a hole, slot and/or depression. More particularly, this invention relates to a method and apparatus for making a hole, slot, and/or depression in a catheter needle proximal to its tip.

### BACKGROUND OF THE INVENTION

Needle safety devices available today employ the hole, slot and/or depression in a needle proximal to its tip to stop the needle guard from tipping over the needle tip. The purpose of having a hole, slot and/or depression in a needle proximal to its tip is to usually allow a needle safety device to lodge into the said hole, slot and/or depression. Therefore, the hole, slot and/or depression acts as a stopping mechanism for a safety device. These safety devices are usually used in catheters and injections. The purpose of having a safety device over a needle is to ensure that the healthcare worker is not pricked or stabbed accidentally with the needle tip while attending a patient which may lead to transmission of diseases compromising the safety of healthcare worker.

Generally, there are numerous ways to drill, cut or create the hole, slot and/or depression in a needle proximal to its tip. For example, the hole may be made by drill machine, laser beam machine, electron beam machine, punch machine and the like. The production of needles by such processes involves many processing steps and different types of machinery to perform each step. The accuracy and precise repeatability of the various processes become more critical in the preparation of high quality needles. Such processes require movement of the body of the needle from one piece of machinery to another as sequential processing step.

Generally, a cutting tool rotates around its own axis while feeding forward into the material being drilled. When the drill reaches the other side of the tube material being drilled, the drill tip bursts through creating ragged edges that spread out to the final sized hole, leaving an exit burr around the hole. Exit burrs are unacceptable in a catheter assembly as they create stress risers and fatigue cracks in the structure. It also generates a lot of heat and thrust force which requires additional power from the drilling machine. Further, the sequential movement steps increases the time and manpower involvement making it a time consuming and expensive affair for needle production.

Another disadvantage of creating a hole in a needle proximal to its tip by conventional drilling is that it compromises the high standard requirement of the accurate and precise drilling where a hole may end up misaligned. With the advent of the technology, medical needles have become increasingly smaller in diameter size, for example to the order of less than a millimeter or even less thus requiring increased precision in needle production techniques. Smaller diameter size makes it more complex and costly to manufacture the medical needles with a hole. The manufacturing of small diameter needles creates handling difficulties in drilling the hole in the body of the needle. The small diameter needle body requires extreme precision to ensure centrality of the drill formed therein. Moreover, decrease in the diameter of the needle proportionally decreases the length of the needle. Precise handling of short, very fine needle body without causing damage to the needle body and to the operator handling the drilling operations is quite difficult to achieve in a high volume production environment.

The precision to ensure centrality of the drill in the body of the needle raises handling difficulties affecting the production of the needles. In the conventional drilling process the needle tube holder which holds the tube causes handling difficulties, and the position of the held tube is not always satisfactory. Also a high degree of manual skill is needed for smooth, reliable and uniform drilling of a syringe needle point. Thus, the result of drilling becomes poor and time consuming with wide variations in the drilling volume depending on the amount of correction, and syringe needles with irregular dimensions are produced. In the case of mass production, it is difficult to obtain products of uniform quality.

Accordingly, there remains a constant need for a commercially viable method and apparatus for making a hole, slot and/or depression in a needle proximal to its tip which addresses the above mentioned disadvantages as well as the need of mass production at a high speed in a cost effective manner.

WO 2004/065036 A1 (p. 21, I. 18 to p. 24, I. 7; figures 20, 21) discloses a grinder device for needle tubes, said grinder device comprising a strip securing in parallel alignment a plurality of needle tubes exposing the tips of the needle tubes, a holding means assembly for the said strip comprising at least two holding plates for clamping there between the said strip, and a grinding wheel assembly having a grinding wheel with a grinding surface positioned adjacent said holding means assembly.

### SUMMARY OF THE INVENTION

Aspects of the present disclosure are set forth in the accompanying independent claims. Optional features of embodiments are set out in the dependent claims.

### BRIEF DESCRIPTION OF THE ACCOMPANYING DRAWINGS

**FIG**. **1** is a perspective view of one embodiment of a grinder device to which the methods of the present invention may be applied;
**FIG. 2** is a front view of one embodiment of a grinder device to which the methods of the present invention may be applied;
**FIG. 3** is a front view of the strip containing plurality of needle tubes positioned adjacent to each other according to a preferred embodiment of the present invention;
**FIGS. 4 (a) and 4 (b)** are perspective views of the needle holding means assembly according to a preferred embodiment of the present invention;

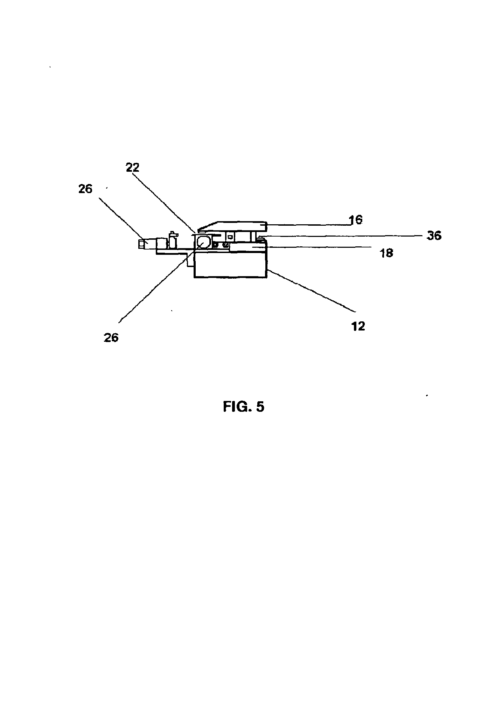

**FIG. 5** is a side view of the needle holding assembly according to a preferred embodiment of the present invention;
**FIG. 6** is a perspective view of the needle holding means assembly being mounted on the movable table of a grinder device according to a preferred embodiment of the present invention;
**FIG. 7** is a front view of the needle holding means assembly being mounted on the movable table of a grinder device according to a preferred embodiment of the present invention;
**FIG. 8** is a perspective view of the grinding wheel assembly positioned adjacent to the needle holding means assembly according to a preferred embodiment of the present invention;
**FIG. 9** is front view of the grinding wheel assembly positioned adjacent to the needle holding means assembly according to a preferred embodiment of the present invention;
**FIG. 10** is a perspective view of one embodiment of a grinder device in operational mode to which the methods of the present invention may be applied;
**FIGS. 11** and **12** are views of the needle tubes before and after grinding according to a preferred embodiment of the present invention.

### DETAILED DESCRIPTION OF THE PREFERRED EMBODIMENTS

Referring to the accompanying drawings, a preferred embodiment of the present invention will now be described. In the Figures, like numerals denote like parts.

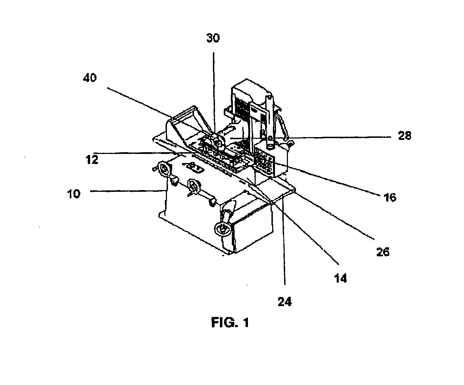

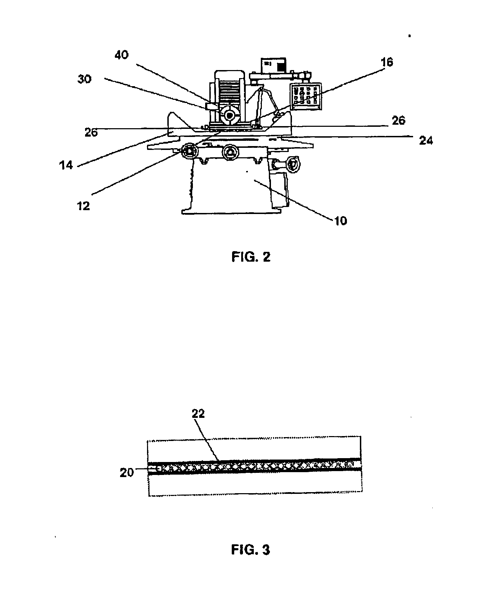

With reference to **Fig. 1**, and as particularly clearly illustrated in **Fig. 2**, a movable table 14 forms a base platform for the holding means assembly 12 comprising needle holding top plate 16 and needle holding bottom plate 18. A strip 22 securing in parallel alignment a plurality of catheter needle tubes 20 is clamped between the needle holding top plate 16 and the needle holding bottom plate 18. The movable table 14 is positioned on the table bed 24.

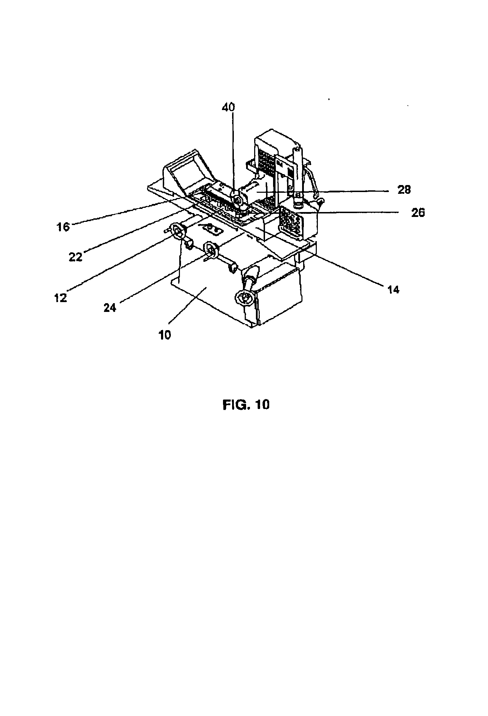

The underside of movable table 14 has therein grooves, not shown in the drawings, and matching ribs formed on the top side of the table bed 24, such that the movable table 14 is free to slide on the table bed 24 in the longitudinal direction. One or plurality of pneumatic cylinders 26 are connected to both the movable table 14 and holding means assembly 12. Limit switches are provided which detect and act when the movable table 14 reaches the end of the table bed 24 as clearly illustrated in **Fig. 10****.** The action of limit switches stops operation of pneumatic cylinders 26 stopping the grinding operation.

Adjacent to the movable table 14 of the grinder device 10 there is provided a grinding wheel assembly 28 comprising a grinding wheel 40 with a grinding surface 30. The grinding wheel 40 is connected via grinding support to a drive motor. As shown, the grinding wheel assembly 28 may comprise a metal core, e.g., stainless steel, provided with an abrasive material, e.g., a diamond/binder mixture, in an outer circumferential recess thereof. Alternatively, the grinding wheel 40 may be provided with any abrasive material such as silicon carbide, diamond, aluminum oxide, etc., as well as mixtures thereof. The support member and the impeller are preferably formed from a metal, such as stainless steel. The grinding wheel 40 is electronically controlled to give the correct and desired depth to create a hole, slot and/or depression 34 as per the specification profile in the exposed tips of the needles housed in the strip 22. During operation, the grinding wheel assembly 28 is rotated by the motor. For creating a hole, slot and/or depression 34 in a needle 20 proximal to its tip, the preferred rotational speed is generally about 2850 RPM. The rotational speed may be varied depending on the use of the various specification sizes of the needle tubes 20.

**Fig. 3** illustrates a strip 22 comprising plurality of needle tubes 20. In the strip 22, needle tubes 20 can be supported in a parallel uniform manner positioned adjacent each other. The needle strip 22 facilitates precision as per the specification required for making a hole, slot and/or depression 34 proximal to the tip 32 of the needle tubes 20. The needle tubes 20 are housed in the strip 22 in a uniform manner exposing the pointed tip region 32 of the needle 20 where a hole, slot and/or depression 34 has to be made. The needle tubes 20 supported on the needle strip 22 after being clamped on the needle holding means assembly 12 stick out exposing the tips 32 of the needles 20 in a perpendicular direction. Once the needles 20 supported on needle strip 22 are placed at the desired length on the holding means assembly 12, the assembly 12 is then mounted on a moveable table 14 of the grinder device 10.

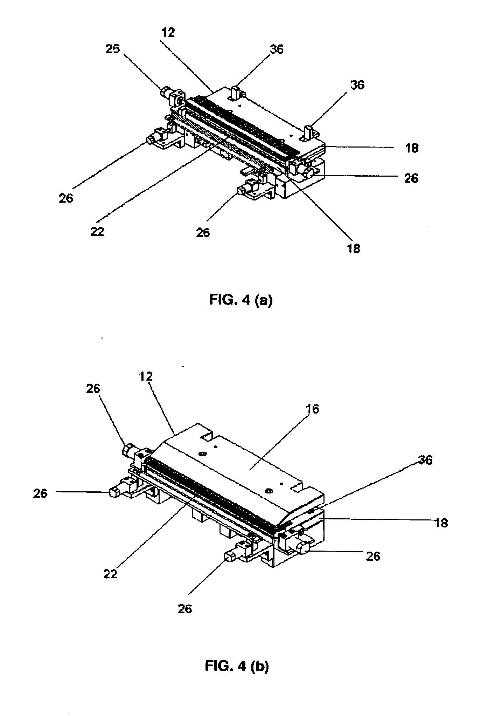

As shown in **Figs. 4 (a),** 4 **(b)** and **5**, the needle holding means assembly 12 comprises a needle holding bottom plate 18 and a needle holding top plate 16 which holds the strip 22 housing plurality of needle tubes 20 clamped in between said two plates 16, 18. One or plurality of pneumatic cylinders 26 control the movement of the holding plates 16, 18 and the clamping operation of the strip 22 in between the holding plates 16, 18. The pneumatic cylinders 26 function in a normal and regular manner as already known. The air pressure for the pneumatic cylinders 26 is controlled by an electronic switch. In this description the holding means assembly 12 shows at least four pneumatic cylinders 26. However, there could be more or less pneumatic cylinders 26 as long as the clamping operation is performed efficiently.

The strip 22 comprising the needles 20 fits within and between the holder space being covered with top and bottom rubber surface cover elements, and is held therebetween and clamped by the forward movement of the angle support block means 36. There are positioned a pair of guide blocks and side blocks which support the strip 22 through the needle holding means assembly 12. The angle support block means 36 control change in the position and thereby the angle at which the tubes 20 supported by strip 22 extend toward grinding surface 30 of the grinding wheel 40. The strip 22 is designed such that, as illustrated in **Fig. 3**, a plurality of needle tubes 20 can be supported in a parallel manner positioned adjacent each other. The needle holding means assembly 12 comprising the needle strip 22 is mounted on the moveable plate 14 of a grinder device 10. During the grinding process, a strip 22 exposing the needle tips 32 is pressed against the grinding surface 30 of rotating grinding wheel 40.

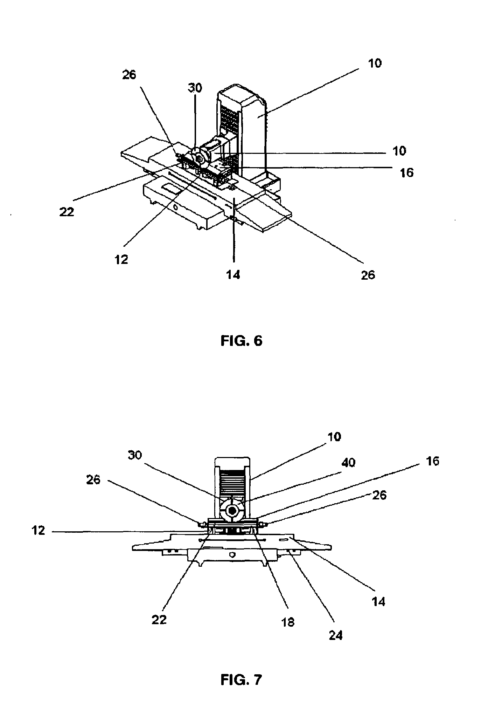

As shown in **Fig. 6** and **7**, the holding means assembly 12 comprising the strip 22 being mounted on the movable table 14 is moved relative to the grinding wheel 40 in a direction generally perpendicular to the axis of the strip 22 exposing the needle tips 32. When in operation, the grinding surface 30 rotates about the tool axis and orbits about the principal axis. The grinding wheel assembly 28 is positioned and secured to the holding means assembly 12 such that the principal axis of the grinding surface 30 is substantially fixed with respect to the holding means assembly 12. When in operation, the grinding surface 30 rotates about the wheel axis and rotates about the principal axis to cut the hole, slot and/or depression 34 in the needle 20 proximal to the pointed tip 32.

Holes/slots 34 are, thus, created in the needle tips 32 by the grinding wheel 40 and is finished with the accurate profile of the hole, slot and/or depression 34 to be created in the needle tip 32. The holding means assembly 12 comprises space adjusting means for separating two holding plates 16, 18 by an adjustable predetermined distance, to thereby enable the holding means assembly 12 to accommodate needle tubes 20 of varying diameters. The holding means assembly 12 also comprises space adjuster means for determining the changes between the positions of the needle tubes 20 arranged in the strip 22 to a pre-determined value for grinding a hole, slot and/or depression 34 in the tip 32 of the needle 20.

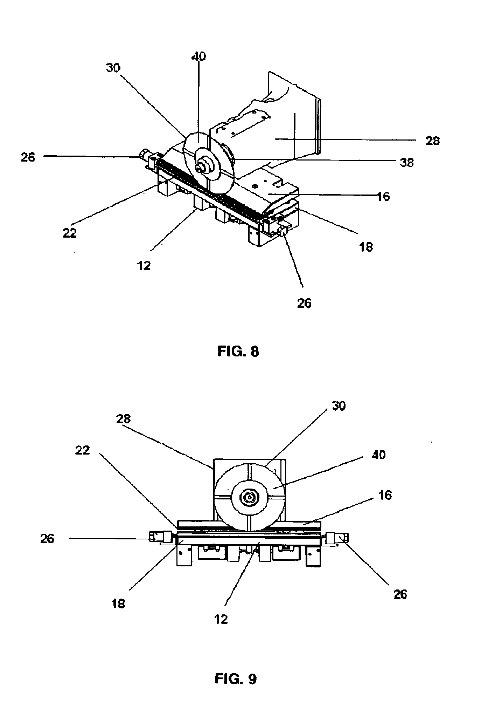

As shown in **Figs. 8** and **9**, the grinding wheel 40 is axially connected to the grinder device 10 with the help of an axel 38. The holding means assembly 12 comprising a strip 22 exposing needle tips 32 being clamped in-between the holding plates 16, 18 extend generally perpendicular to the axis of the strip 22. The holding means assembly 12 comprising the strip 22 is adjusted under the grinding surface 30 of the grinding wheel 40. The space adjusting means maintains the spacing between the needle tips 32 exposed in the strip 22 and the plane of the grinding surface 30 of the grinding wheel 40. **Figs. 8** and **9** also show the positioning of the grinding wheel 40 on the needle holding means assembly 12. The operator then electronically adjusts the height of the holding means assembly 12 so that the grinding wheel 40 creates the hole, slot and/or depression 34 of the desired specification in the exposed needle 20 tips 32. The holding means assembly 12 comprises means for clamping the said strip 22.

After the height is fixed, the operator starts the grinding wheel 40 and starts the moveable table 14 holding the holding means assembly 12. The holding means assembly 12 starts moving towards the rotating grinding wheel 40. As the exposed tips 32 of the needles 20 supported in the strip 22 come in touch with the grinding wheel 40, it creates a hole, slot and/or depression 34 on the tip 32 of the needle of desired profile specification. By the time the holding means assembly 12 reaches the other end of the moveable table, it results in the creation of holes/slots 34 on the needles 20 supported in the strip 22.
This method of creating the needles 20 with holes, slots and or depressions 34 take less than about 5 minutes. The number of needles 20 that can be loaded on the needle strip 22 can be as less as about 250 in numbers and vary in numbers to the extent of about 1000 needle tubes 20 depending upon the outer diameter of the needle tubes 20.

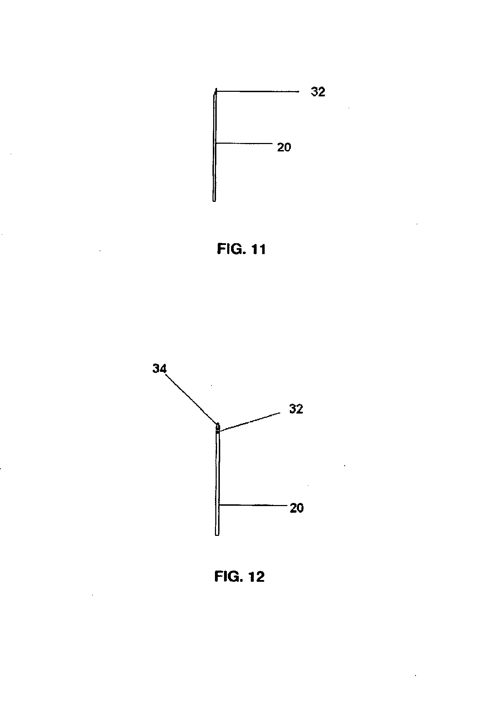

**Fig. 11** depicts a stainless steel tube 20 conforming to the standard requirements for producing catheter needles. The depicted tube 20 with a pointed tip 32 is drawn through a die to a specified inner and a specified outer diameter. This stainless steel tube 20 is cut to an adequate length as per the specification profile. After the burrs are removed from the cut section of the cut tube 20, the cut tubes 20 are routinely washed and dried.

This needle 20 before grinding is usually of the size required for an injection or a catheter. For the illustrative purpose of this description the specification size of the needle tubes 20 is 54.8 mm long and 0.55 mm thick. However, the present invention encompasses usage of the needle tubes 20 of various specification and sizes.

**Fig. 12** shows a needle tube 20 after a hole, slot and/or depression 34 being grinded on the needle proximal to its tip 32 as per a pre-determined specification.

The advantage of the method, as described above, is that over about 400 needle tubes 20 with a hole, slot and/or depression 34 can be created in a time frame which is taken actually for one needle 20 and thereby saving time, effort and money. Also, there is no need for intervention of a skilled worker in mid-process, thereby reducing substantially the manpower requirements. Also, a large number of catheter needles 20 can be produced to the same specification with a remarkably increased efficiency by the implementation of the above disclosed apparatus and method.

The present invention also solves previous difficulties as discussed in the prior art which impeded the automation of grinding operations to create hole, slot and/or depression 34 in the needle 20 and assures the accurate automatic grinding of holes/slots 34 in the needle 20.

Although, the invention has been described with reference to specific examples, it would be appreciated by those skilled in the art that the invention may be embodied in many forms without departing from the scope of the invention as set forth in the invention. Thus, variations of preferred embodiments as disclosed may become apparent to those of ordinary skill in the art upon reading the foregoing description. The specification and drawings, therefore, are to be regarded in an illustrative rather than a restrictive manner without departing from the scope of the invention, the invention resides in the claims hereinafter appended.

### LIST OF REFERENCE NUMERALS

- 10: grinder device
- 12: needle holding means assembly
- 14: movable table
- 16: needle holding top plate
- 18: needle holding bottom plate
- 20: needle tube
- 22: strip
- 24: table bed
- 26: pneumatic cylinders
- 28: grinding wheel assembly
- 30: grinding surface
- 32: pointed needle tip
- 34: hole, slot and/or depression
- 36: angle support block means
- 38: axel
- 40: grinding wheel

## Claims

1. A grinder device (10) for making a hole, slot and/or depression (34) in a needle tube (20) proximal to its tip (32), said grinder device (10) comprising:
a strip (22) securing in parallel alignment a plurality of needle tubes (20) exposing the tips (32) of the needle tubes (20);
a holding means assembly (12) for the said strip (22) comprising at least two holding plates (16,18) for clamping therebetween the said strip (22) wherein portions of said holding means assembly (12) which contact the said strip (22) are provided with friction resistance rubber surface cover elements, and wherein said holding means assembly (12) comprises angle support block means (36) for changing the position and thereby the angle at which the needle tubes (20) supported by said strip (22) extend toward a grinding surface (30) of a grinding wheel (40);
a pair of guide blocks and side blocks to support the said strip (22) through the said holding means assembly (12);
a grinding wheel assembly (28), having said grinding wheel (40) with said grinding surface (30), positioned adjacent said holding means assembly (12);
a movable table (14) operably mounted on the grinder device (10) configured to move the said holding means assembly (12) therealong for changing the position of the needle tubes (20) supported in the said strip (22) by said holding means assembly (12).

2. The grinder device (10) according to claim 1, wherein said holding means assembly (12) comprises space adjusting means for separating said two holding plates (16,18) by an adjustable predetermined distance, to thereby enable the holding means assembly (12) to accommodate needle tubes (20) of varying diameter.

3. The grinder device (10) according to claim 1, wherein said holding means assembly (12) comprises space adjuster means for determining the changes between the position of the needle tubes (20) arranged in the said strip (22) to a pre-determined value for grinding a hole, slot and/or depression (34) in the tip (32) of the needle tube (20).

4. The grinder device (10) according to claim 1, wherein said holding means assembly (12) comprises means for clamping the said strip (22).

5. The grinder device (10) according to claim 1, wherein said holding means assembly (12) comprises at least one pneumatic cylinder (26).

6. The grinder device (10) according to claim 1, wherein the holding means assembly (12) comprises a needle holding top plate (16) and a needle holding bottom plate (18) for securing the said strip (22) in-between.

7. The grinder device (10) according to any one of the preceding claims, further comprising grinding control means for controlling the sequence of operation of the above-mentioned said means.

8. A method for making a hole, slot and/or depression (34) in a needle tube (20) proximal to its tip (32), the method comprising the steps of:
positioning a plurality of needle tubes (20) in parallel alignment in a strip (22);
clamping the said strip (22) between a holding means assembly (12) comprising at least two holding plates (16,18) wherein portions of said holding means assembly (12) which contact the said strip (22) are provided with friction resistance rubber surface cover elements, and wherein said holding means assembly (12) comprises angle support block means (36) for changing the position and thereby the angle at which the needle tubes (20) supported by said strip (22) extend toward a grinding surface (30) of a grinding wheel (40);
supporting the strip (22) through the holding means assembly (12) with a pair of guide blocks and side blocks;
mounting the said holding means assembly (12) on a movable table (14) of the grinder device (10);
positioning a grinding wheel assembly (28), having said grinding wheel (40) with said grinding surface (30), adjacent to the said holding means assembly (12);
bringing in contact the strip (22) exposing the needle tip (32) of the plurality of needle tubes (20) to the grinding surface (30) of the said grinding wheel assembly (28);
grinding a hole, slot and/or depression (34) in a needle tube (20) proximal to its tip (32) as per a pre-determined specification; and
extracting, washing and drying the needle tubes (20).

9. The method according to claim 8, wherein said strip (22) is clamped in between the holding means assembly (12) such that the needle tip (32) is exposed generally within a perpendicular area projected by the remainder of needle tube (20) being clamped un-exposed in-between the said holding plates (16,18).

## Patentansprüche

1. Schleifvorrichtung (10) zum Herstellen eines Lochs, eines Schlitzes und/oder einer Vertiefung (34) in einem Nadelrohr (20) proximal zu seiner Spitze (32), wobei die Schleifvorrichtung (10) aufweist:
einen Streifen (22) der eine Vielzahl von Nadelrohren (20) in paralleler Ausrichtung festlegt, wobei die Spitzen (32) der Nadelrohre (20) freiliegen;
eine Haltemittelanordnung (12) für den Streifen (22), die wenigstens zwei Halteplatten (16, 18) aufweist, um den Streifen (22) dazwischen einzuklemmen, wobei Abschnitte der Haltemittelanordnung (12), die den Streifen (22) berühren, mit Reibungswiderstands-Abdeckelementen mit Gummioberfläche versehen sind, und wobei die Haltemittelanordnung (12) ein Winkelstützblockmittel (36) zum Ändern der Position und damit des Winkels, in dem sich die von dem Streifen (22) gehaltenen Nadelrohre (20) in Richtung einer Schleiffläche (30) eines Schleifrads (40) erstrecken, aufweist;
ein Paar von Führungsblöcken und Seitenblöcken zum Halten des Streifens (22) durch die Haltemittelanordnung (12);
eine Schleifradanordnung (28), welche das Schleifrad (40) mit der Schleiffläche (30) aufweist, und welche nahe der Haltemittelanordnung (12) angeordnet ist;
einen beweglichen Tisch (14), der betriebsbereit an der Schleifvorrichtung (40) montiert ist und dazu konfiguriert ist, die Haltemittelanordnung (12) dort entlang zu bewegen, um die Position der durch die Haltemittelanordnung (12) in dem Streifen (22) gehaltenen Nadelrohre (20) zu verändern.

2. Schleifvorrichtung (10) nach Anspruch 1, wobei die Haltemittelanordnung (12) Abstandseinstellmittel zum Trennen der beiden Halteplatten (16, 18) um einen einstellbaren vorgegebenen Abstand aufweist, um dadurch zu ermöglichen, dass die Haltemittelanordnung (12) Nadelrohre (20) unterschiedlichen Durchmessers aufnimmt.

3. Schleifvorrichtung (10) nach Anspruch 1, wobei die Haltemittelanordnung (12) Abstandseinstellmittel zum Festlegen der Änderungen zwischen den Positionen der in dem Streifen (22) angeordneten Nadelrohre (20) auf einen vorgegebenen Wert zum Schleifen eines Lochs, eines Schlitzes und/oder einer Vertiefung (34) in der Spitze (32) des Nadelrohrs aufweist.

4. Schleifvorrichtung (10) nach Anspruch 1, wobei die Haltemittelanordnung (12) Mittel zum Klemmen des Streifens (22) aufweist.

5. Schleifvorrichtung (10) nach Anspruch 1, wobei die Haltemittelanordnung (12) wenigstens einen Pneumatikzylinder (26) aufweist.

6. Schleifvorrichtung (10) nach Anspruch 1, wobei die Haltemittelanordnung (12) eine Nadelhalte-Oberplatte (16) und eine Nadelhalte-Bodenplatte (18) zum Sichern des Streifens (22) dazwischen aufweist.

7. Schleifvorrichtung (10) nach einem der vorhergehenden Ansprüche, die des Weiteren ein Schleifsteuermittel zum Steuern der Betriebsabfolge der oben genannten Mittel aufweist.

8. Verfahren zum Herstellen eines Lochs, eines Schlitzes und/oder einer Vertiefung (34) in einem Nadelrohr (20) proximal zu seiner Spitze (32), wobei das Verfahren die folgenden Schritte umfasst:
Positionieren einer Vielzahl von Nadelrohren (20) in paralleler Ausrichtung in einem Streifen (22);
Klemmen des Streifens (22) zwischen einer Haltemittelanordnung (12), die wenigstens zwei Halteplatten (16, 18) aufweist, wobei Abschnitte der Haltemittelanordnung (12), die den Streifen (22) berühren, mit Reibungswiderstands-Abdeckelementen mit Gummioberfläche versehen sind, und wobei die Haltemittelanordnung (12) ein Winkelstützblockmittel (36) zum Ändern der Position und damit des Winkels, in dem sich die von dem Streifen (22) gehaltenen Nadelrohre (20) in Richtung einer Schleiffläche (30) eines Schleifrads (40) erstrecken, aufweist;
Halten des Streifens (22) durch die Haltemittelanordnung (12) mit einem Paar von Führungsblöcken und Seitenblöcken;
Anbringen der Haltemittelanordnung (12) auf einem beweglichen Tisch (14) der Schleifvorrichtung (10);
Positionieren einer Schleifradanordnung (28), die das Schleifrad (40) mit der Schleiffläche (30) aufweist, nahe der Haltemittelanordnung (12);
Inkontaktbringen des Streifens (22) der Vielzahl von Nadelrohren (20) mit freiliegender Nadelspitze (32) mit der Schleiffläche (30) der Schleifradanordnung (28);
Schleifen eines Lochs, eines Schlitzes und/oder einer Vertiefung (34) in einem Nadelrohr (20) proximal zu seiner Spitze (32) gemäß einer vorgegebenen Spezifikation; und
Herausziehen, Waschen und Trocknen der Nadelrohre (20).

9. Verfahren nach Anspruch 8, wobei der Streifen (22) zwischen der Haltemittelanordnung (12) derart geklemmt wird, dass die Nadelspitze (32) im Allgemeinen in einem senkrechten Bereich freiliegt, der vom Rest des Nadelrohrs (20) projiziert wird, welcher nicht freiliegend zwischen den Halteplatten (16, 18) geklemmt wird.

## Revendications

1. Dispositif de meuleuse (10) pour réaliser un trou, une fente et/ou une dépression (34) dans un tube d'aiguille (20) à proximité de sa pointe (32), ledit dispositif de meuleuse (10) comprenant :
une bande (22) fixant en alignement parallèle une pluralité de tubes d'aiguille (20) exposant les pointes (32) des tubes d'aiguille (20) ;
un ensemble de moyens de maintien (12) pour ladite bande (22) comprenant au moins deux plaques de maintien (16, 18) pour serrer entre elles ladite bande (22), des parties dudit ensemble de moyens de maintien (12) qui sont en contact avec ladite bande (22) étant munies d'éléments de revêtement de surface en caoutchouc à résistance au frottement, et ledit ensemble de moyens de maintien (12) comprenant des moyens de bloc de support d'angle (36) pour changer la position et ainsi l'angle auquel les tubes d'aiguille (20) supportés par ladite bande (22) s'étendent vers une surface de meulage (30) d'une meule (40) ;
une paire de blocs de guidage et de blocs latéraux pour supporter ladite bande (22) à travers ledit ensemble de moyens de maintien (12) ;
un ensemble meule (28) ayant ladite meule (40) avec ladite surface de meulage (30), positionné adjacent audit ensemble de moyens de maintien (12) ;
une table mobile (14) montée de façon fonctionnelle sur le dispositif de meuleuse (10), configurée pour déplacer ledit ensemble de moyens de maintien (12) le long de celui-ci pour changer la position des tubes d'aiguille (20) supportés dans ladite bande (22) par ledit ensemble de moyens de maintien (12).

2. Dispositif de meuleuse (10) selon la revendication 1, dans lequel ledit ensemble de moyens de maintien (12) comprend des moyens d'ajustement d'espace pour séparer lesdites deux plaques de maintien (16, 18) d'une distance prédéterminée ajustable, pour permettre ainsi à l'ensemble de moyens de maintien (12) de recevoir des tubes d'aiguille (20) de diamètre variable.

3. Dispositif de broyeur (10) selon la revendication 1, dans lequel ledit ensemble de moyens de maintien (12) comprend des moyens d'ajustement d'espace pour déterminer les changements entre la position des tubes d'aiguille (20) agencés dans ladite bande (22) à une valeur prédéterminée pour meuler un trou, une fente et/ou une dépression (34) dans la pointe (32) du tube d'aiguille (20).

4. Dispositif de meuleuse (10) selon la revendication 1, dans lequel ledit ensemble de moyens de maintien (12) comprend des moyens pour serrer ladite bande (22).

5. Dispositif de meuleuse (10) selon la revendication 1, dans lequel ledit ensemble de moyens de maintien (12) comprend au moins un vérin pneumatique (26).

6. Dispositif de meuleuse (10) selon la revendication 1, dans lequel l'ensemble de moyens de maintien (12) comprend une plaque supérieure de maintien d'aiguille (16) et une plaque inférieure de maintien d'aiguille (18) pour fixer ladite bande (2) entre elles.

7. Dispositif de meuleuse (10) selon l'une quelconque des revendications précédentes, comprenant en outre des moyens de commande de meulage pour commander la séquence de fonctionnement desdits moyens mentionnés ci-dessus.

8. Procédé pour réaliser un trou, une fente et/ou une dépression (34) dans un tube d'aiguille (20) à proximité de sa pointe (32), le procédé comprenant les étapes consistant à :
positionner une pluralité de tubes d'aiguille (20) en alignement parallèle dans une bande (22) ;
serrer ladite bande (22) entre un ensemble de moyens de maintien (12) comprenant au moins deux plaques de maintien (16, 18), des parties dudit ensemble de moyens de maintien (12) qui sont en contact avec ladite bande (22) étant munies d'éléments de revêtement de surface en caoutchouc à résistance au frottement, et ledit ensemble de moyens de maintien (12) comprenant des moyens de bloc de support d'angle (36) pour changer la position et ainsi l'angle auquel les tubes d'aiguille (20) supportés par ladite bande (22) s'étendent vers une surface de meulage (30) d'une meule (40) ;
supporter la bande (22) à travers l'ensemble de moyens de maintien (12) avec une paire de blocs de guidage et de blocs latéraux ;
monter ledit ensemble de moyens de maintien (12) sur une table mobile (14) du dispositif de meuleuse (10) ;
positionner un ensemble meule (28), ayant ladite meule (40) avec ladite surface de meulage (30), adjacent audit ensemble de moyens de maintien (12) ;
amener la bande (22) exposant la pointe d'aiguille (32) de la pluralité de tubes d'aiguille (20) en contact avec la surface de meulage (30) dudit ensemble meule (28) ;
meuler un trou, une fente et/ou une dépression (34) dans un tube d'aiguille (20) à proximité de sa pointe (32) conformément à une spécification prédéterminée ; et
extraire, laver et sécher les tubes d'aiguille (20).

9. Procédé selon la revendication 8, dans lequel ladite bande (102) est serrée entre l'ensemble de moyens de maintien (12) de telle sorte que la pointe d'aiguille (32) est exposée généralement au sein d'une zone perpendiculaire projetée par le reste du tube d'aiguille (20) qui est serré de façon non exposée entre lesdites plaques de maintien (16, 18).
